# EUROPEAN PATENT APPLICATION

(11) **EP 4 194 470 A1**
(43) Date of publication of application: **14.06.2023**
(21) Application number: 22207465.0
(22) Date of filing: 18.10.2018
(51) Int. Cl.: C07K 16/28, A61P 35/00, A61K 39/395

(54) **TREATMENT OF OVARIAN CANCER WITH ANTI-CD47 AND ANTI-PD-L1**

(30) Priority: 18.10.2017 US 201762574073 P
(62) Divisional of application: 18807145.0
(71) Applicant: Forty Seven, Inc., Foster City, CA 94404 (US)
(72) Inventor: TAKIMOTO, Chris Hidemi, Mizufune, Foster City, 94404 (US); VOLKMER, Jens-Peter, Foster City, 94404 (US); CHAO, Mark, Ping, Foster City, 94404 (US)
(74) Representative: Carpmaels & Ransford LLP

(57) **Abstract**

Methods are provided for treating individuals with ovarian cancers with an anti-CD47 antibody and an anti PD-L1 antibody.

## Description

### CROSS REFERENCE TO RELATED APPLICATIONS

This application claims benefit of U.S. Provisional Application No. 62/574,073, filed October 18, 2017, which is hereby incorporated in its entirety by reference for all purposes.

### SEQUENCE LISTING

The instant application contains a Sequence Listing which has been submitted electronically in ASCII format and is hereby incorporated by reference in its entirety. Said ASCII copy, created on September 26, 2018, is named 41404WO_CRF_sequencelisting.txt and is 12,403 bytes in size.

### BACKGROUND

Ovarian cancer is the most frequent cause of gynecological cancer deaths in the USA. Standard systemic therapy for newly diagnosed patients with advanced disease typically involves the use of platinum-based chemotherapy with or without antiangiogenic agents. More recently targeted agents, such as PARP inhibitors, have been utilized in women with advanced disease and BRCA mutations. However, newer immunotherapies, such as the checkpoint inhibitors, have not impacted clinical outcomes sufficiently to become standard of care. In women with recurrent ovarian cancer, the platinum-free interval (PFI), defined as the interval between the last platinum dose and the date of relapse is strongly correlated with the likelihood of response to subsequent chemotherapy. Patients with a PFI greater than 1 month but less than 6 months were historically referred to as platinum-resistant, and these patients have had limited treatment options. Thus, the development of effective cancer treatments for these patients would address a substantial unmet medical need.

The immune system's natural capacity to detect and destroy abnormal cells may prevent the development of many cancers. However, cancer cells are sometimes able to avoid detection and destruction by the immune system. Cancer cells can reduce the expression of tumor antigens on their surface, making it harder for the immune system to detect them; express proteins on their surface that induce immune cell inactivation; and/or induce cells in the microenvironment to release substances that suppress immune responses and promote tumor cell proliferation and survival.

Cancer immunotherapies have been developed to enhance immune responses against tumors, by stimulating specific components of the immune system; or by counteracting signals produced by cancer cells that suppress immune responses.

One approach blocks immune checkpoint proteins, which limit the strength and duration of immune responses. These proteins normally keep immune responses in check by preventing overly intense responses that might damage normal cells as well as abnormal cells. Blocking the activity of immune checkpoint proteins releases the "brakes" on the immune system, increasing its ability to destroy cancer cells.

Immune checkpoint inhibitors in current clinical use include ipilimumab, which blocks the activity of CTLA4, which is expressed on the surface of activated cytotoxic T lymphocytes. CTLA4 acts as a "switch" to inactivate these T cells, thereby reducing the strength of immune responses; inhibiting it increases the cytotoxic T cell response. Two other FDA-approved checkpoint inhibitors, nivolumab and pembrolizumab work in a similar way, but they target PD-1. A third FDA-approved checkpoint inhibitor, Avelumab, targets PD-L1.

Other forms of immunotherapy use proteins that normally help regulate, or modulate, immune system activity to enhance the body's immune response against cancer, e.g. interleukins and interferons. Antibodies targeted to tumor cell antigens are also in clinical use.

Some forms of immunotherapy exploit the innate immune system. The cell surface protein CD47 on healthy cells and its engagement of a phagocyte receptor, SIRPalpha, constitutes a key "don't eat-me" signal that can turn off engulfment mediated by multiple modalities, including apoptotic cell clearance and FcR mediated phagocytosis. Blocking the CD47 mediated engagement of SIRPalpha on a phagocyte, or the loss of CD47 expression in knockout mice, can cause removal of live cells and non-aged erythrocytes. Alternatively, blocking SIRPalpha recognition also allows engulfment of targets that are not normally phagocytosed. Anti-CD47 antibody treatment has also been shown to not only enable macrophage phagocytosis of cancer, but can also initiate an anti-tumor cytotoxic T cell immune response.

Combinations of immune regulatory agents with CD47 blockade can also enhance efficacy of the immune regulatory agents by promoting tumor antigen presentation and depletion of inhibitory immune cells. This enables a shortening of treatment period and thus reduces the duration and significance of potential toxicities and side effects.

Related publications include "Engineered Sirp alpha Variants Asm Immunotherapeutic Adjuvants To Anticancer Antibodies." Science 341(6141): 88-91; Willingham, S. B., J. P. Volkmer, Et Al. (2012). "The Cd47-Signal Regulatory Protein Alpha (Sirpa) Interaction Is A Therapeutic Target For Human Solid Tumors." Proc Natl Acad Sci U S A 109(17): 6662-6667. Chao, M. P., A. A. Alizadeh, Et Al. (2010). "Anti-Cd47 Antibody Synergizes With Rituximab To Promote Phagocytosis And Eradicate Non-Hodgkin Lymphoma." Cell 142(5): 699-713. Boyerinas B, Jochems C, Fantini M, Heery CR, Gulley JL, Tsang, KY, and Schlom J. Antibody-Dependent Cellular Cytotoxicity Activity of a Novel Anti-PD-L1 Antibody Avelumab (MSB0010718C) on Human Tumor Cells Cancer Immunol Res 2015;3(10): 1148-57. Davis A, Tinker AV, Friedlander M. "Platinum resistant" ovarian cancer: What is it, who to treat and how to measure benefit? Gynecol Oncol 2014;133:624-631. Disis ML, Patel MR, Pant S, Hamilton EP, Lockart AC, Kelly K, Beck JT, Gordon MS, Weiss, GJ, Taylor MH, Chaves J, Mita AC, Chin KM, von Heydebreck A, , Cuillerot J-M, Gulley JL. Avelumab (MSB0010718C; anti-PD-L1) in patients with recurrent/refractory ovarian cancer from the JAVELIN Solid Tumor phase 1b trial: Safety and clinical activity. J Clin Oncol 34, 2016 (suppl; abstr 5533). Rustin GJS, Vergote I, Eisenhauer E, et al. Definitions for Response and Progression in Ovarian Cancer Clinical Trials Incorporating RECIST 1.1 and CA 125 Agreed by the Gynecological Cancer Intergroup (GCIG). Int J Gynecol Cancer 2011;21: 419-423. Seymour L, Bogaerts J, Perrone A, et al. RECIST working group. iRECIST: guidelines for response criteria for use in trials testing immunotherapeutics. Lancet Oncol. 2017 Mar;18(3):e143-e152. Tseng D, Volkmer J-P, Willingham SB, et al., Anti-CD47 antibody-mediated phagocytosis of cancer by macrophages primes an effective antitumor. PNAS 2013;110(27):11103-11108. Wilson MK 1, Pujade-Lauraine E, Aoki D, et al. Fifth Ovarian Cancer Consensus Conference of the Gynecologic Cancer InterGroup: recurrent disease. Annals of Oncol 2017; 28: 727-732. Yanagita T, Murata Y, Tanaka D, et al, Anti-SIRPa antibodies as a potential new tool for cancer immunotherapy. JCI Insight, 2017;2(1):e89140. Related applications include: Methods for Achieving Therapeutically Effective Doses of anti-CD47 Agents for Treating Cancer, US Pat. No. 9,623,079. Treatment of cancer with combinations of immunoregulatory agents, US Patent Application No. US 15/411,623, each of which is herein incorporated by reference, in its entirety, for all purposes.

### SUMMARY

Methods are provided for treating an individual, e.g., a human subject, with a therapeutic combination of an anti-CD47 antibody and an anti-PD-L1 antibody. A benefit of the present invention can be the use of lowered doses of the agents, e.g., the anti-CD47 antibody and the anti-PD-L1 antibody, relative to the dose required as a single immunoregulatory agent, or a combination of immunoregulatory agents in the absence of CD47 blockade. A benefit of the present invention can also, or alternatively, be a decrease in the length of time required for treatment, relative to the length of time required for treatment as a single immunoregulatory agent, or a combination of immunoregulatory agents in the absence of CD47 blockade. A benefit of the present invention can also, or alternatively, be an enhanced response relative to the response observed after treatment with a single immunoregulatory agent, or a combination of immunoregulatory agents in the absence of CD47 blockade.

The methods of the invention comprise administration of an anti-CD47 antibody. In some embodiments the antibody comprises a human IgG4 Fc region. In some embodiments the anti-CD47 antibody competes for binding to CD47 with Hu5F9-G4. In some embodiments, the anti-CD47 antibody binds to the same CD47 epitope as Hu5F9-G4. In other embodiments, the anti-CD47 antibody is Hu5F9-G4.

The methods of the invention comprise administration of an anti-PD-L1 antibody. In some embodiments, the anti-PD-L1 antibody is Avelumab (Bavencio ^{®}).

In some embodiments, the anti-CD47 antibody is Hu5F9-G4 and the anti-PD-L1 antibody is Avelumab (Bavencio ^{®})

In some embodiments, the ovarian cancer is an epithelial ovarian cancer, optionally serous tumor, mucinous tumor, clear cell tumor, endometriod tumor, transitional cell tumor, Brenner tumor, carcinosarcoma tumor, mixed epithelial tumor, borderline epithelial tumor, undifferentiated carcinoma tumor, fallopian tube tumor, or primary peritoneal tumor. In some embodiments, the epithelial ovarian cancer is serous tumor, e.g., the serous tumor ovarian cancer is low grade or high grade as determined by histological analysis subtyping. In some embodiments, the tumor type is determined by histological analysis.

In some embodiments, the subject is anti-PD-L1 antibody naive. The subject can be platinum sensitive or, alternatively, platinum resistant.

The methods of the present invention comprise administration of the anti-CD47 antibody and/or the anti-PD-L1 antibody by any appropriate delivery. In some embodiments, the anti-CD47 antibody and/or the anti-PD-L1 antibody is administered intra-abdominally. In some embodiments, the anti-CD47 antibody and/or the anti-PD-L1 antibody is administered intra-tumorally. In some embodiments, the anti-CD47 antibody and/or the anti-PD-L1 antibody is administered intravenously. The anti-CD47 antibody and the anti-PD-L1 antibody can be administered concurrently or sequentially

In some embodiments of the invention, administration of the anti-CD47 antibody and/or the anti-PD-L1 antibody reduce the level of cancer markers such as CA125, HE4 (human epididymis protein 4), CA-72-4, CA-19-9, and CEA; compared to baseline. In some embodiments, administration of the anti-CD47 antibody and/or the anti-PD-L1 antibody reduce CA125 in the subject compared to baseline. In some embodiments, the level of CA125 is measured about once per month. In other embodiments, administration reduces the level of CA125 in the subject by at least 30-90, 40-80, 50-70, 30, 40, 50, 60, 70, 80, or 90% compared to baseline. In other embodiments, administration reduces the size of the cancer or metastases thereof compared to baseline, optionally as measured by imaging, optionally wherein the imaging is CT/PET/CT or MRI, optionally comprising disease that increases initially from baseline but subsequently decreases in size.

In some embodiment a therapeutic regimen for treatment of cancer comprises administration of a loading dose an anti-CD47 antibody, including without limitation 5F9-G4, where the loading dose is administered twice weekly at a dose of from 20 mg/kg to 67.5 mg/kg; and may be administered twice weekly at a dose of from 20 mg/kg to 30 mg/kg. The patient is then administered a maintenance dose, weekly or semi-weekly, at a dose of from 10 mg/kg to 40 mg/kg; and may be at a dose of from 20 mg/kg to 30 mg/kg. In some such embodiments the cancer is an ovarian cancer. In some such embodiments the cancer is an epithelial ovarian cancer e.g. a serous tumor, mucinous tumor, clear cell tumor, endometriod tumor, transitional cell tumor, Brenner tumor, carcinosarcoma tumor, mixed epithelial tumor, borderline epithelial tumor, undifferentiated carcinoma tumor, fallopian tube tumor, or primary peritoneal tumor.

In some embodiments, the therapeutically effective amount of the anti-PD-L1 antibody is 10 mg/kg. In some embodiments, the anti-PD-L1 antibody is administered every 14 days. In some embodiments, the anti-PD-L1 antibody is administered 7 days after the priming dose and every 14 days thereafter. In some embodiments, the anti-PD-L1 antibody is administered on the same day as the priming dose and every 14 days thereafter.

The present invention also includes a composition comprising an anti-CD47 antibody and an anti-PD-L1 antibody. The present invention also includes a kit comprising an anti-CD47 antibody, an anti-PD-L1 antibody, and instructions for use.

### DETAILED DESCRIPTION

Methods are provided for the treatment of ovarian cancer in a subject or reducing the size of the ovarian cancer, the treatment comprising administering to the subject an anti-CD47 antibody and an anti-PD-L1 antibody.

Before the present active agents and methods are described, it is to be understood that this invention is not limited to the particular methodology, products, apparatus and factors described, as such methods, apparatus and formulations may, of course, vary. It is also to be understood that the terminology used herein is for the purpose of describing particular embodiments only, and is not intended to limit the scope of the present invention which will be limited only by appended claims.

It must be noted that as used herein and in the appended claims, the singular forms "a," "and," and "the" include plural referents unless the context clearly dictates otherwise. Thus, for example, reference to "a drug candidate" refers to one or mixtures of such candidates, and reference to "the method" includes reference to equivalent steps and methods known to those skilled in the art, and so forth.

Unless defined otherwise, all technical and scientific terms used herein have the same meaning as commonly understood by one of ordinary skill in the art to which this invention belongs. All publications mentioned herein are incorporated herein by reference for the purpose of describing and disclosing devices, formulations and methodologies which are described in the publication and which might be used in connection with the presently described invention.

Where a range of values is provided, it is understood that each intervening value, to the tenth of the unit of the lower limit unless the context clearly dictates otherwise, between the upper and lower limit of that range and any other stated or intervening value in that stated range is encompassed within the invention. The upper and lower limits of these smaller ranges may independently be included in the smaller ranges is also encompassed within the invention, subject to any specifically excluded limit in the stated range. Where the stated range includes one or both of the limits, ranges excluding either both of those included limits are also included in the invention.

In the following description, numerous specific details are set forth to provide a more thorough understanding of the present invention. However, it will be apparent to one of skill in the art that the present invention may be practiced without one or more of these specific details. In other instances, well-known features and procedures well known to those skilled in the art have not been described in order to avoid obscuring the invention.

Generally, conventional methods of protein synthesis, recombinant cell culture and protein isolation, and recombinant DNA techniques within the skill of the art are employed in the present invention. Such techniques are explained fully in the literature, see, e.g., Maniatis, Fritsch & Sambrook, Molecular Cloning: A Laboratory Manual (1982); Sambrook, Russell and Sambrook, Molecular Cloning: A Laboratory Manual (2001); Harlow, Lane and Harlow, Using Antibodies: A Laboratory Manual: Portable Protocol No. I, Cold Spring Harbor Laboratory (1998); and Harlow and Lane, Antibodies: A Laboratory Manual, Cold Spring Harbor Laboratory; (1988).

### Definitions

As used herein, an "anti-CD47 antibody" refers to any antibody that reduces the binding of CD47 (e.g., on a target cell) to SIRPα (e.g., on a phagocytic cell). Non-limiting examples are described in more detail below and include but are not limited to Hu5F9-G4.

As described in more detail below, an anti-PD-L1 antibody is an antibody that inhibits binding of the PD-L1 (PD1 ligand) to PD1 (programmed death 1). Examples include avelumab.

As used herein, "antibody" includes reference to an immunoglobulin molecule immunologically reactive with a particular antigen, and includes both polyclonal and monoclonal antibodies. The term also includes genetically engineered forms such as chimeric antibodies (e.g., humanized murine antibodies) and heteroconjugate antibodies. The term "antibody" also includes antigen binding forms of antibodies, including fragments with antigen-binding capability (*e.g*., Fab', F(ab')₂, Fab, Fv and rIgG. The term also refers to recombinant single chain Fv fragments (scFv). The term antibody also includes bivalent or bispecific molecules, diabodies, triabodies, and tetrabodies. Additional description of the term antibody is found below.

A "patient" for the purposes of the present invention includes both humans and other animals, particularly mammals, including pet and laboratory animals, e.g. mice, rats, rabbits, *etc.* Thus the methods are applicable to both human therapy and veterinary applications. In one embodiment the patient is a mammal, preferably a primate. In other embodiments the patient is human.

The terms "subject," "individual," and "patient" are used interchangeably herein to refer to a mammal being assessed for treatment and/or being treated. In an embodiment, the mammal is a human. The terms "subject," "individual," and "patient" encompass, without limitation, individuals having cancer. Subjects may be human, but also include other mammals, particularly those mammals useful as laboratory models for human disease, *e.g.* mouse, rat, *etc.*

As used herein, the phrase "platinum sensitive" refers to a human subject that develops recurrent disease greater than 6 months after receiving the last platinum-based chemotherapy.

As used herein, the phrase "platinum resistant" refers to a human subject that develops recurrent disease less than 6 months after receiving the last platinum-based chemotherapy.

As used herein, the term "baseline' is defined as a 30 day period prior to first treatment administration to human subject with ovarian cancer.

The term "sample" with respect to a patient encompasses blood and other liquid samples of biological origin, solid tissue samples such as a biopsy specimen or tissue cultures or cells derived therefrom and the progeny thereof. The definition also includes samples that have been manipulated in any way after their procurement, such as by treatment with reagents; washed; or enrichment for certain cell populations, such as cancer cells. The definition also includes sample that have been enriched for particular types of molecules, e.g., nucleic acids, polypeptides, *etc.* The term "biological sample" encompasses a clinical sample, and also includes tissue obtained by surgical resection, tissue obtained by biopsy, cells in culture, cell supernatants, cell lysates, tissue samples, organs, bone marrow, blood, plasma, serum, and the like. A "biological sample" includes a sample obtained from a patient's cancer cell, *e.g.,* a sample comprising polynucleotides and/or polypeptides that is obtained from a patient's cancer cell (*e.g.,* a cell lysate or other cell extract comprising polynucleotides and/or polypeptides); and a sample comprising cancer cells from a patient. A biological sample comprising a cancer cell from a patient can also include non-cancerous cells.

The term "diagnosis" is used herein to refer to the identification of a molecular or pathological state, disease or condition, such as the identification of a molecular subtype of breast cancer, prostate cancer, or other type of cancer.

The term "prognosis" is used herein to refer to the prediction of the likelihood of cancer-attributable death or progression, including recurrence, metastatic spread, and drug resistance, of a neoplastic disease, such as ovarian cancer. The term "prediction" is used herein to refer to the act of foretelling or estimating, based on observation, experience, or scientific reasoning. In one example, a physician may predict the likelihood that a patient will survive, following surgical removal of a primary tumor and/or chemotherapy for a certain period of time without cancer recurrence.

As used herein, the terms "treatment," "treating," and the like, refer to administering an agent, or carrying out a procedure, for the purposes of obtaining an effect. The effect may be prophylactic in terms of completely or partially preventing a disease or symptom thereof and/or may be therapeutic in terms of effecting a partial or complete cure for a disease and/or symptoms of the disease. "Treatment," as used herein, may include treatment of a tumor in a mammal, particularly in a human, and includes: (a) preventing the disease or a symptom of a disease from occurring in a subject which may be predisposed to the disease but has not yet been diagnosed as having it (*e.g*., including diseases that may be associated with or caused by a primary disease; (b) inhibiting the disease, i.e., arresting its development; and (c) relieving the disease, *i.e.,* causing regression of the disease.

Treating may refer to any indicia of success in the treatment or amelioration or prevention of an cancer, including any objective or subjective parameter such as abatement; remission; diminishing of symptoms or making the disease condition more tolerable to the patient; slowing in the rate of degeneration or decline; or making the final point of degeneration less debilitating. The treatment or amelioration of symptoms can be based on objective or subjective parameters; including the results of an examination by a physician. Accordingly, the term "treating" includes the administration of the compounds or agents of the present invention to prevent or delay, to alleviate, or to arrest or inhibit development of the symptoms or conditions associated with cancer or other diseases. The term "therapeutic effect" refers to the reduction, elimination, or prevention of the disease, symptoms of the disease, or side effects of the disease in the subject.

"In combination with", "combination therapy" and "combination products" refer, in certain embodiments, to the concurrent administration to a patient of the agents described herein. When administered in combination, each component can be administered at the same time or sequentially in any order at different points in time. Thus, each component can be administered separately but sufficiently closely in time so as to provide the desired therapeutic effect.

"Concomitant administration" of active agents in the methods of the invention means administration with the reagents at such time that the agents will have a therapeutic effect at the same time. Such concomitant administration may involve concurrent (*i.e.* at the same time), prior, or subsequent administration of the agents. A person of ordinary skill in the art would have no difficulty determining the appropriate timing, sequence and dosages of administration for particular drugs and compositions of the present invention.

As used herein, the term "correlates," or "correlates with," and like terms, refers to a statistical association between instances of two events, where events include numbers, data sets, and the like. For example, when the events involve numbers, a positive correlation (also referred to herein as a "direct correlation") means that as one increases, the other increases as well. A negative correlation (also referred to herein as an "inverse correlation") means that as one increases, the other decreases.

"Dosage unit" refers to physically discrete units suited as unitary dosages for the particular individual to be treated. Each unit can contain a predetermined quantity of active compound(s) calculated to produce the desired therapeutic effect(s) in association with the required pharmaceutical carrier. The specification for the dosage unit forms can be dictated by (a) the unique characteristics of the active compound(s) and the particular therapeutic effect(s) to be achieved, and (b) the limitations inherent in the art of compounding such active compound(s).

A "therapeutically effective amount" means the amount that, when administered to a subject for treating a disease, is sufficient to effect treatment for that disease.

### Methods of Treatment

Methods are provided for treating a human subject having ovarian cancer or reducing the size of the ovarian cancer, the method comprising administering to the subject an anti-CD47 antibody and an anti-PD-L1 antibody. Such methods include administering to a subject in need of treatment a therapeutically effective amount or an effective dose of the combined agents of the invention, including without limitation combinations with an ESA.

Anti-PD-L1 antibodies can enhance the efficacy of anti-CD47 antibodies. The anti-CD47 antibody can be administered in combination or prior to the anti-PD-L1 antibody to simulate priming of tumor-specific T cells that can expand if the inhibitory anti-PD1/PD-L1 pathway is blocked.

A combination of an anti-CD47 antibody with an anti-PD-L1 antibody described herein is given to patients with tumors subtypes that are responsive to these therapies. These tumors may be defined by a higher frequency of mutations, resulting in more tumor antigens, therefore being more immunogenic, as described herein. In some embodiments patients treated with combination therapy are responsive to treatment with an immune activator or checkpoint inhibitor; however this represents a subset of approximately 25% of patients within a specific potentially responsive tumor subtype. In some embodiments, the individuals may be platinum therapy sensitive or resistant.

In some embodiments, the subject methods include a step of administering a primer agent to subject, followed by a step of administering a therapeutically effective dose of an anti-CD47 antibody and an anti-PD-L1 antibody to the subject. In some embodiments, the step of administering a therapeutically effective dose is performed after at least about 3 days (e.g., at least about 4 days, at least about 5 days, at least about 6 days, at least about 7 days, at least about 8 days, at least about 9 days, or at least about 10 days) after beginning the administration of a primer agent. This period of time is, for example, sufficient to provide for enhanced reticulocyte production by the individual.

The administration of a therapeutically effective dose of an anti-CD47 antibody and/or an anti-PD-L1 antibody can be achieved in a number of different ways. In some cases, two or more therapeutically effective doses are administered after a primer agent is administered. Suitable administration of a therapeutically effective dose can entail administration of a single dose, or can entail administration of doses daily, semi-weekly, weekly, once every two weeks, once a month, annually, *etc.* In some cases, a therapeutically effective dose is administered as two or more doses of escalating concentration (i.e., increasing doses), where (i) all of the doses are therapeutic doses, or where (ii) a sub-therapeutic dose (or two or more sub-therapeutic doses) is initially given and therapeutic doses are achieved by said escalation. As one non-limiting example to illustrate escalating concentration (i.e., increasing doses), a therapeutically effective dose can be administered weekly, beginning with a sub-therapeutic dose (e.g., a dose of 5 mg/kg), and each subsequent dose can be increased by a particular increment (e.g., by 5 mg/kg), or by variable increments, until a therapeutic dose (e.g., 30 mg/kg) is reached, at which point administration may cease or may continue (e.g., continued therapeutic doses, e.g., doses of 30 mg/kg). As another non-limiting example to illustrate escalating concentration (i.e., increasing doses), a therapeutically effective dose can be administered weekly, beginning with a therapeutic dose (e.g., a dose of 10 mg/kg), and each subsequent dose can be increased by a particular increment (e.g., by 10 mg/kg), or by variable increments, until a therapeutic dose (e.g., 30 mg/kg, 100 mg/ml, etc.) is reached, at which point administration may cease or may continue (e.g., continued therapeutic doses, e.g., doses of 30 mg/kg, 100 mg/ml, etc.). In some embodiments, administration of a therapeutically effective dose can be a continuous infusion and the dose can altered (e.g., escalated) over time.

Dosage and frequency may vary depending on the half-life of the anti-CD47 antibody and/or the anti-PD-L1 antibody in the patient. It will be understood by one of skill in the art that such guidelines will be adjusted for the molecular weight of the active agent, *e.g.* in the use of antibody fragments, in the use of antibody conjugates, in the use of soluble CD47 peptides etc. The dosage may also be varied for localized administration, e.g. intranasal, inhalation, *etc.,* or for systemic administration, *e.g.* i.m., i.p., i.v., s.c., and the like.

In certain embodiments of the invention, the anti-CD47 antibody is infused to a patient in an initial dose, and optionally in subsequent doses, over a period of time and/or concentration that reduces the possibility of hematologic microenvironments where there is a high local concentration of RBC and the agent.

In some embodiments of the invention, an initial dose of the anti-CD47 antibody is infused over a period of at least about 2 hours, at least about 2.5 hours, at least about 3 hours, at least about 3.5 hours, at least about 4 hours, at least about 4.5 hours, at least about 5 hours, at least about 6 hours or more. In some embodiments an initial dose is infused over a period of time from about 2.5 hours to about 6 hours; for example from about 3 hours to about 4 hours. In some such embodiments, the dose of agent in the infusate is from about 0.05 mg/ml to about 0.5 mg/ml; for example from about 0.1 mg/ml to about 0.25 mg/ml.

The subject methods also include the co-administration of an anti-PD-L1 antibody with the anti-CD47 antibody. In some embodiments, the anti-PD-L1 antibody is Avelumab. In some embodiments, the individual receiving the treatment is anti-PD-L1 antibody naive. The anti-PD-L1 antibody may be administered in together with the anti-CD47 antibody or separately, in any appropriate delivery method, e.g. i.v., i.p., subcutaneously, intra-tumorally, or intra-abdominally. The therapeutically effective amount of the anti-PD-L1 antibody may be about 10 mg/kg. In some embodiments, the anti-PD-L1 antibody may be administered every 14 days. In other embodiments, the anti-PD-L1 antibody may be administered 7 days after the priming does of the anti-CD47 antibody and every 14 days thereafter. In other embodiments, the anti-PD-L1 antibody may be administered with the priming does of the anti-CD47 antibody and every 14 days thereafter.

### Ovarian Cancer

Provided herein are methods for treating individuals having an ovarian cancer or reducing the size of the ovarian cancer in the subject, comprising administering: a therapeutically effective amount of an anti-CD47 antibody to the subject; and a therapeutically effective amount of at least one anti-PD-L1 antibody to the subject.

Examples of ovarian cancer include epithelial ovarian cancer, optionally serous tumor, mucinous tumor, clear cell tumor, endometriod tumor, transitional cell tumor, Brenner tumor, carcinosarcoma tumor, mixed epithelial tumor, borderline epithelial tumor, undifferentiated carcinoma tumor, fallopian tube tumor, or primary peritoneal tumor.

In some embodiments, the epithelial ovarian cancer is serous tumor. The serous tumor ovarian cancer can be determined to be low grade or high grade by histological analysis subtyping. In one embodiment, the individuals are platinum chemotherapy sensitive. In another embodiment, the individuals are platinum chemotherapy resistant. In some embodiments, the individuals are PD-L1 naïve.

In some embodiments, the patient has a low mutation burden. In some embodiments, the patent has a high mutation burden. As is known in the art, cancer types can vary in the average or specific degree of mutation, where higher levels of mutation are associated with increased expression of neoantigens. See, for example, Vogelstein et al., (2013), supra. A low mutation burden can be a cancer type with an average per tumor, or specific number for an individual tumor, of up to about 10, up to about 20, up to about 30, up to about 40, up to about 50 non-synonymous mutations per tumor. A high mutation burden can be a cancer type with greater than about 50, greater than about 75, greater than about 100, greater than about 125, greater than about 150 non-synonymous mutations per tumor.

In some such embodiments the cancer is, without limitation, ovarian cancer. In some such embodiments, the cancer is a type that has a high neoantigen, or mutagenesis, burden (see Vogelstein et al. (2013) Science 339(6127): 1546-1558, herein specifically incorporated by reference). In other embodiments, the cancer with a type with a low neoantigen burden. In some such embodiments, the combination therapy of the present invention enhances the activity of the checkpoint inhibitor. In other embodiments, where the individual cancer does not respond to a checkpoint inhibitor alone, the combination therapy provides a therapeutic response. In some embodiments, the individual is platinum sensitive. In other embodiments, the individual is platinum resistant.

### Cancer

The terms "cancer," "neoplasm," and "tumor" are used interchangeably herein to refer to cells which exhibit autonomous, unregulated growth, such that they exhibit an aberrant growth phenotype characterized by a significant loss of control over cell proliferation. Cells of interest for detection, analysis, or treatment in the present application include precancerous (*e.g.,* benign), malignant, pre-metastatic, metastatic, and non-metastatic cells. Cancers of virtually every tissue are known. The phrase "cancer burden" refers to the quantum of cancer cells or cancer volume in a subject. Reducing cancer burden accordingly refers to reducing the number of cancer cells or the cancer volume in a subject. The term "cancer cell" as used herein refers to any cell that is a cancer cell or is derived from a cancer cell e.g. clone of a cancer cell. Many types of cancers are known to those of skill in the art, including solid tumors such as carcinomas, sarcomas, glioblastomas, melanomas, lymphomas, myelomas, etc., and circulating cancers such as leukemias. Examples of cancer include but are not limited to, ovarian cancer, breast cancer, colon cancer, lung cancer, prostate cancer, hepatocellular cancer, gastric cancer, pancreatic cancer, cervical cancer, ovarian cancer, liver cancer, bladder cancer, cancer of the urinary tract, thyroid cancer, renal cancer, carcinoma, melanoma, head and neck cancer, and brain cancer.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, neoplasia, premalignancy, malignancy, invasion of surrounding or distant tissues or organs, such as lymph nodes, *etc.*

As used herein, the terms "cancer recurrence" and "tumor recurrence," and grammatical variants thereof, refer to further growth of neoplastic or cancerous cells after diagnosis of cancer. Particularly, recurrence may occur when further cancerous cell growth occurs in the cancerous tissue. "Tumor spread," similarly, occurs when the cells of a tumor disseminate into local or distant tissues and organs; therefore tumor spread encompasses tumor metastasis. "Tumor invasion" occurs when the tumor growth spread out locally to compromise the function of involved tissues by compression, destruction, or prevention of normal organ function.

As used herein, the term "metastasis" refers to the growth of a cancerous tumor in an organ or body part, which is not directly connected to the organ of the original cancerous tumor. Metastasis will be understood to include micrometastasis, which is the presence of an undetectable amount of cancerous cells in an organ or body part which is not directly connected to the organ of the original cancerous tumor. Metastasis can also be defined as several steps of a process, such as the departure of cancer cells from an original tumor site, and migration and/or invasion of cancer cells to other parts of the body.

### Clinical endpoints

The methods described herein result in at least one improved endpoint compared to baseline.

In some embodiments of the invention, administration of the anti-CD47 antibody and/or the anti-PD-L1 antibody reduce the level of cancer markers such as CA125, HE4 (human epididymis protein 4), CA-72-4, CA-19-9, and CEA; compared to baseline. In some embodiments, administration of the anti-CD47 antibody and/or the anti-PD-L1 antibody reduce CA125 in the subject compared to baseline. In some embodiments, the level of CA125 is measured about once per month. In other embodiments, administration reduces the level of CA125 in the subject by at least 30-90, 40-80, 50-70, 30, 40, 50, 60, 70, 80, or 90% compared to baseline. CA125 can be measured with an immunoassay. CA125 can be measured using one or more of the assays disclosed in Mongia et al., Performance characteristics of seven automated CA 125 assays. Am J Clin Pathol. 2006 Jun; 125(6):921-7; herein incorporated by reference for all purposes. In other embodiments, administration reduces the size of the cancer or metastases thereof compared to baseline, optionally as measured by imaging, optionally wherein the imaging is CT/PET/CT or MRI, optionally comprising disease that increases initially from baseline but subsequently decreases in size.

As used herein, endpoints for treatment will be given a meaning as known in the art and as used by the Food and Drug Administration.

Overall survival is defined as the time from randomization until death from any cause, and is measured in the intent-to-treat population. Survival is considered the most reliable cancer endpoint, and when studies can be conducted to adequately assess survival, it is usually the preferred endpoint. This endpoint is precise and easy to measure, documented by the date of death. Bias is not a factor in endpoint measurement. Survival improvement should be analyzed as a risk-benefit analysis to assess clinical benefit. Overall survival can be evaluated in randomized controlled studies. Demonstration of a statistically significant improvement in overall survival can be considered to be clinically significant if the toxicity profile is acceptable, and has often supported new drug approval. A benefit of the methods of the invention can include increased overall survival of patients.

Endpoints that are based on tumor assessments include DFS, ORR, TTP, PFS, and time-to-treatment failure (TTF). The collection and analysis of data on these time-dependent endpoints are based on indirect assessments, calculations, and estimates (e.g., tumor measurements). Disease-Free Survival (DFS) is defined as the time from randomization until recurrence of tumor or death from any cause. The most frequent use of this endpoint is in the adjuvant setting after definitive surgery or radiotherapy. DFS also can be an important endpoint when a large percentage of patients achieve complete responses with chemotherapy.

Objective Response Rate. ORR is defined as the proportion of patients with tumor size reduction of a predefined amount and for a minimum time period. Response duration usually is measured from the time of initial response until documented tumor progression. Generally, the FDA has defined ORR as the sum of partial responses plus complete responses. When defined in this manner, ORR is a direct measure of drug antitumor activity, which can be evaluated in a single-arm study.

Time to Progression and Progression-Free Survival. TTP and PFS have served as primary endpoints for drug approval. TTP is defined as the time from randomization until objective tumor progression; TTP does not include deaths. PFS is defined as the time from randomization until objective tumor progression or death. The precise definition of tumor progression is important and should be carefully detailed in the protocol.

### Antibodies

The methods described herein include administration of an antibody or antibodies, i.e., administration of an anti CD47 antibody and, in some embodiments, administration of an anti PD-L1 antibody. As described above, the term "antibody" includes reference to an immunoglobulin molecule immunologically reactive with a particular antigen, and includes both polyclonal and monoclonal antibodies. The term also includes genetically engineered forms such as chimeric antibodies (e.g., humanized murine antibodies) and heteroconjugate antibodies. The term "antibody" also includes antigen binding forms of antibodies, including fragments with antigen-binding capability (*e.g*., Fab', F(ab')₂, Fab, Fv and rIgG. The term also refers to recombinant single chain Fv fragments (scFv). The term antibody also includes bivalent or bispecific molecules, diabodies, triabodies, and tetrabodies.

Selection of antibodies may be based on a variety of criteria, including selectivity, affinity, cytotoxicity, *etc.* The phrase "specifically (or selectively) binds" to an antibody or "specifically (or selectively) immunoreactive with," when referring to a protein or peptide, refers to a binding reaction that is determinative of the presence of the protein, in a heterogeneous population of proteins and other biologics. Thus, under designated immunoassay conditions, the specified antibodies bind to a particular protein sequences at least two times the background and more typically more than 10 to 100 times background. In general, antibodies of the present invention bind antigens on the surface of target cells in the presence of effector cells (such as natural killer cells or macrophages). Fc receptors on effector cells recognize bound antibodies.

An antibody immunologically reactive with a particular antigen can be generated by recombinant methods such as selection of libraries of recombinant antibodies in phage or similar vectors, or by immunizing an animal with the antigen or with DNA encoding the antigen. Methods of preparing polyclonal antibodies are known to the skilled artisan. The antibodies may, alternatively, be monoclonal antibodies. Monoclonal antibodies may be prepared using hybridoma methods. In a hybridoma method, an appropriate host animal is typically immunized with an immunizing agent to elicit lymphocytes that produce or are capable of producing antibodies that will specifically bind to the immunizing agent. Alternatively, the lymphocytes may be immunized *in vitro.* The lymphocytes are then fused with an immortalized cell line using a suitable fusing agent, such as polyethylene glycol, to form a hybridoma cell.

Human antibodies can be produced using various techniques known in the art, including phage display libraries. Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire.

Antibodies also exist as a number of well-characterized fragments produced by digestion with various peptidases. Thus pepsin digests an antibody below the disulfide linkages in the hinge region to produce F(ab)'₂, a dimer of Fab which itself is a light chain joined to V_{H}-C_{H1} by a disulfide bond. The F(ab)'₂ may be reduced under mild conditions to break the disulfide linkage in the hinge region, thereby converting the F(ab)'₂ dimer into an Fab' monomer. The Fab' monomer is essentially Fab with part of the hinge region. While various antibody fragments are defined in terms of the digestion of an intact antibody, one of skill will appreciate that such fragments may be synthesized *de novo* either chemically or by using recombinant DNA methodology. Thus, the term antibody, as used herein, also includes antibody fragments either produced by the modification of whole antibodies, or those synthesized *de novo* using recombinant DNA methodologies (e.g., single chain Fv) or those identified using phage display libraries.

A "humanized antibody" is an immunoglobulin molecule which contains minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, a humanized antibody will comprise substantially all of at least one, and typically two, variable domains, in which all or substantially all of the CDR regions correspond to those of a non-human immunoglobulin and all or substantially all of the framework (FR) regions are those of a human immunoglobulin consensus sequence. The humanized antibody optimally also will comprise at least a portion of an immunoglobulin constant region (Fc), typically that of a human immunoglobulin.

Antibodies of interest may be tested for their ability to induce ADCC (antibody-dependent cellular cytotoxicity) or ADCP (antibody dependent cellular phagocytosis). Antibody-associated ADCC activity can be monitored and quantified through detection of either the release of label or lactate dehydrogenase from the lysed cells, or detection of reduced target cell viability (e.g. annexin assay). Assays for apoptosis may be performed by terminal deoxynucleotidyl transferase-mediated digoxigenin-11-dUTP nick end labeling (TUNEL) assay (Lazebnik et al., Nature: 371, 346 (1994). Cytotoxicity may also be detected directly by detection kits known in the art, such as Cytotoxicity Detection Kit from Roche Applied Science (Indianapolis, Ind.).

### CD47 Antibodies

The methods described herein include administration of an anti-CD47 antibody.

CD47 is a broadly expressed transmembrane glycoprotein with a single Ig-like domain and five membrane spanning regions, which functions as a cellular ligand for SIRPalpha with binding mediated through the NH2-terminal V-like domain of SIRPalpha. SIRPalpha is expressed primarily on myeloid cells, including macrophages, granulocytes, myeloid dendritic cells (DCs), mast cells, and their precursors, including hematopoietic stem cells. Structural determinants on SIRPalpha that mediate CD47 binding are discussed by Lee et al. (2007) J. Immunol. 179:7741-7750; Hatherley et al. (2008) Mol Cell. 31(2):266-77; Hatherley et al. (2007) J.B.C. 282:14567-75; and the role of SIRPalpha cis dimerization in CD47 binding is discussed by Lee et al. (2010) J.B.C. 285:37953-63. In keeping with the role of CD47 to inhibit phagocytosis of normal cells, there is evidence that it is transiently upregulated on hematopoietic stem cells (HSCs) and progenitors just prior to and during their migratory phase, and that the level of CD47 on these cells determines the probability that they are engulfed in vivo.

In some embodiments, the subject anti-CD47 antibody specifically binds CD47 and reduces the interaction between CD47 on one cell (e.g., an infected cell) and SIRPα on another cell (e.g., a phagocytic cell). In some embodiments, a suitable anti-CD47 antibody does not activate CD47 upon binding. Some anti-CD47 antibodies do not reduce the binding of CD47 to SIRPα and such an antibody can be referred to as a "non-blocking anti-CD47 antibody." A suitable anti-CD47 antibody that is an "anti-CD47 agent" can be referred to as a "CD47-blocking antibody". Non-limiting examples of suitable antibodies include clones B6H12, 5F9, 8B6, and C3 (for example as described in International Patent Publication WO 2011/143624, herein specifically incorporated by reference). Suitable anti-CD47 antibodies include fully human, humanized or chimeric versions of such antibodies. Humanized antibodies (e.g., hu5F9-G4) are especially useful for in vivo applications in humans due to their low antigenicity. Similarly caninized, felinized, etc. antibodies are especially useful for applications in dogs, cats, and other species respectively. Antibodies of interest include humanized antibodies, or caninized, felinized, equinized, bovinized, porcinized, etc., antibodies, and variants thereof.

In some embodiments an anti-CD47 antibody comprises a human IgG Fc region, e.g. an IgG1, IgG2a, IgG2b, IgG3, IgG4 constant region. In one embodiment the IgG Fc region is an IgG4 constant region. The IgG4 hinge may be stabilized by the amino acid substitution S241P (see Angal et al. (1993) Mol. Immunol. 30(1): 105-108, herein specifically incorporated by reference).

In some embodiments, the anti-CD47 antibody competes for binding to CD47 with Hu5F9-G4. In some embodiments, the anti-CD47 binds to the same CD47 epitope as Hu5F9-G4.

In some embodiments, the methods described herein include administration of the anti-CD47 antibody Hu5f9-G4. This antibody has been described in US patent 9,623,079, herein specifically incorporated by reference. In some embodiments, the methods described herein include administration of an anti-CD47 antibody with sequences (light chain, heavy chain and/or CDR) at least 97%, at least 98%, at least 99% or 100% identical to the sequences of Hu5f9-G4. Table 1 contains the sequence of the Hu5f9-G4 antibody heavy and light chains. The CDR regions are shown in bold.

**Table 1.**

| SEQ ID NO | Description and Sequence |
|---|---|
| 1 | Hu5f9-G4 Antibodyt Heavy Chain |
| | QVQLVQSGAEVKKPGASVKVSCKASGYTFTNYNMHWVRQA PGQRLEWMGTIYPGNDDTSYNQKFKDRVTITADTSASTAYM ELSSLRSEDTAVYYCARGGYRAMDYWGQGTLVTVSSASTK GPSVFPLAPCSRSTSESTAALGCLVKDYFPEPVTVSWNSGALT SGVHTFPAVLQSSGLYSLSSVVTVPSSSLGTKTYTCNVDHKPS NTKVDKRVESKYGPPCPPCPAPEFLGGPSVFLFPPKPKDTLMI SRTPEVTCVVVDVSQEDPEVQFNWYVDGVEVHNAKTKPREE QFNSTYRVVSVLTVLHQDWLNGKEYKCKVSNKGLPSSIEKTI SKAKGQPREPQVYTLPPSQEEMTKNQVSLTCLVKGFYPSDIA VEWESNGQPENNYKTTPPVLDSDGSFFLYSRLTVDKSRWQEG NVFSCSVMHEALHNHYTQKSLSLSLGK |
| 2 | Hu5f9-G4 Antibodyt Heavy Chain |
| | DIVMTQSPLSLPVTPGEPASISCRSSQSIVYSNGNTYLGWYLQ KPGQSPQLLIYKVSNRFSGVPDRFSGSGSGTDFTLKISRVEAE DVGVYYCFQGSHVPYTFGQGTKLEIKRTVAAPSVFIFPPSDE QLKSGTASVVCLLNNFYPREAKVQWKVDNALQSGNSQESVT EQDSKDSTYSLSSTLTLSKADYEKHKVYACEVTHQGLSSPVT KSFNRGEC |

### PD-L1 Antibodies

The methods described herein include administration of an anti-PD-L1 antibody.

PD-L1 (programmed death ligand 1) is a ligand for PD1. Both PD-L1 and PD1 are examples of an immune checkpoint protein. Immune checkpoint proteins are immune inhibitory molecules that act to decrease immune responsiveness toward a target cell, particularly against a tumor cell in the methods of the invention. Endogenous responses to tumors by T cells can be dysregulated by tumor cells activating immune checkpoints (immune inhibitory proteins) and inhibiting co-stimulatory receptors (immune activating proteins). The class of therapeutic agents referred to in the art as "immune checkpoint inhibitors" reverses the inhibition of immune responses through administering antagonists of inhibitory signals. Other immunotherapies administer agonists of immune costimulatory molecules to increase responsiveness.

Two immune-checkpoint proteins are PD1 and PD-L1. The major role of PD1 is to limit the activity of T cells in peripheral tissues at the time of an inflammatory response to infection and to limit autoimmunity. PD1 expression is induced when T cells become activated. When engaged by one of its ligands, PD1 inhibits kinases that are involved in T cell activation. PD1 is highly expressed on T_{Reg} cells, where it may enhance their proliferation in the presence of ligand. Because many tumors are highly infiltrated with T_{Reg} cells, blockade of the PD1 pathway may also enhance antitumor immune responses by diminishing the number and/or suppressive activity of intratumoral T_{Reg} cells.

The two ligands for PD1 are PD1 ligand 1 (PD-L1; also known as B7-H1 and CD274) and PD-L2 (also known as B7-DC and CD273). The PD1 ligands are commonly upregulated on the tumor cell surface from many different human tumors. On cells from solid tumors, the major PD1 ligand that is expressed is PD-L1. PD-L1 is expressed on cancer cells and through binding to its receptor PD1 on T cells it inhibits T cell activation/function. Therefore, PD1 and PD-L1 blocking agents can overcome this inhibitory signaling and maintain or restore anti-tumor T cell function. Anti-CD47 agents can stimulate a specific anti-tumor T cell response (Anti-CD47 antibody-mediated phagocytosis of cancer by macrophages primes an effective antitumor T-cell response; Tseng et al., Proc Natl Acad Sci U S A. 2013 Jul 2;110(27): 11103-8.)

PD-L1 is expressed on cancer cells and through binding to its receptor PD1 on T cells it inhibits T cell activation/function. Therefore, PD1 and PD-L1 blocking agents can overcome this inhibitory signaling and maintain or restore anti-tumor T cell function. However, since PD-L1 is expressed on tumor cells, antibodies that bind and block PD-L1 can also enable ADCP, ADCC, and CDC of tumor cells. Anti-CD47 agents can synergize with targeted monoclonal antibodies and enhance their potency to stimulate ADCP and ADCC (Anti-CD47 antibody synergizes with rituximab to promote phagocytosis and eradicate non-Hodgkin lymphoma, Chao et al., Cell. 2010 Sep 3;142(5):699-713.) Thus a combination of anti-PD-L1 agents with anti-CD47 agents can enhance the anti-tumor potency. These agents may be administered together (over the same course of treatment, not necessarily the same day and frequency).

Antibodies in current clinical use against PD-L1 include atezolizumab, durvalumab, and avelumab. Avelumab is a human programmed death-ligand 1 (PD-L1) blocking antibody with an active Fc-component (Boyerinas, 2015) approved in the USA for the treatment of locally advanced or metastatic urothelial carcinoma patients who have disease progression during or following platinum-containing chemotherapy, or within 12 months receiving of neoadjuvant or adjuvant platinum-containing chemotherapy. It is also approved for use in adult and pediatric patients 12 years and older with metastatic Merkel cell carcinoma. Avelumab blocks PD-L1/PD-1 mediated inhibition of the adaptive immune response which leads to a T cell directed anti-tumor response.

In some embodiments, the methods described herein include administration of an anti-PD-L1 antibody, e.g., Avelumab. In some embodiments, the methods described herein include administration of an anti-PD-L1 antibody with sequences (light chain, heavy chain and/or CDR) at least 97%, at least 98%, at least 99% or 100% identical to the sequences of Avelumab. Table 2 contains the sequence of the Avelumab antibody heavy and light chains.

**Table 2.**

| SEQ ID NO | Description and Sequence |
|---|---|
| 3 | Avelumab Antibody Heavy Chain |
| | EVQLLESGGGLVQPGGSLRLSCAASGFTFSSYIMMWVRQAPGK GLEWVSSIYPSGGITFYADTVKGRFTISRDNSKNTLYLQMNSLRA EDTAVYYCARIKLGTVTTVDYWGQGTLVTVSSASTKGPSVFPLA PSSKSTSGGTAALGCLVKDYFPEPVTVSWNSGALTSGVHTFPAV LQSSGLYSLSSVVTVPSSSLGTQTYICNVNHKPSNTKVDKKVEPK SCDKTHTCPPCPAPELLGGPSVFLFPPKPKDTLMISRTPEVTCVV VDVSHEDPEVKFNWYVDGVEVHNAKTKPREEQYNSTYRVVSV LTVLHQDWLNGKEYKCKVSNKALPAPIEKTISKAKGQPREPQVY TLPPSRDELTKNQVSLTCLVKGFYPSDIAVEWESNGQPENNYKT TPPVLDSDGSFFLYSKLTVDKSRWQQGNVFSCSVMHEALHNHY TQKSLSLSPGK |
| 4 | Avelumab Antibody Light chain |
| | QSALTQPASVSGSPGQSITISCTGTSSDVGGYNYVSWYQQHPGK APKLMIYDVSNRPSGVSNRFSGSKSGNTASLTISGLQAEDEADY YCSSYTSSSTRVFGTGTKVTVLGQPKANPTVTLFPPSSEELQANK ATLVCLISDFYPGAVTVAWKADGSPVKAGVETTKPSKQSNNKY AASSYLSLTPEQWKSHRSYSCQVTHEGSTVEKTVAPTECS |

### Dosing

The methods described herein include administration of a therapeutically effective dose of compositions, i.e., a therapeutically effective dose of each of an anti-CD47 antibody and an anti-PD-L1 antibody.

Compositions are administered to a patient in an amount sufficient to substantially ablate targeted cells, as described above. An amount adequate to accomplish this is defined as a "therapeutically effective dose", which may provide for an improvement in overall survival rates. Single or multiple administrations of the compositions may be administered depending on the dosage and frequency as required and tolerated by the patient. The particular dose required for a treatment will depend upon the medical condition and history of the mammal, as well as other factors such as age, weight, gender, administration route, efficiency, etc.

Effective doses of the combined agents of the present invention for the treatment of cancer vary depending upon many different factors, including means of administration, target site, physiological state of the patient, whether the patient is human or an animal, other medications administered, and whether treatment is prophylactic or therapeutic. Usually, the patient is a human, but nonhuman mammals may also be treated, e.g. companion animals such as dogs, cats, horses, *etc.,* laboratory mammals such as rabbits, mice, rats, *etc.,* and the like. Treatment dosages can be titrated to optimize safety and efficacy.

A therapeutically effective dose of the anti-CD47 antibody can depend on the specific agent used, but is usually about 20 mg/kg body weight or more (e.g., about 20 mg/kg or more, about 25 mg/kg or more, about 30 mg/kg or more, about 35 mg/kg or more, about 40 mg/kg or more, about 45 mg/kg or more, about 50 mg/kg or more, or about 55 mg/kg or more, or about 60 mg/kg or more, or about 65 mg/kg or more, or about 70 mg/kg or more), or from about 20 mg/kg to about 70 mg/kg (e.g., from about 20 mg/kg to about 67.5 mg/kg, or from about 20 mg/kg to about 60 mg/kg).

In some embodiments, the therapeutically effective dose of the anti-CD47 antibody is 20, 30, 45, 60, or 67.5 mg/kg. In some embodiments, the therapeutically effective dose of the anti-CD47 antibody is 20 to 60 mg/kg. In some embodiments, the therapeutically effective dose of the anti-CD47 antibody is 20 to 67.5 mg/kg.

A therapeutically effective dose of the anti-PD-L1 antibody can depend on the specific antibody used, but is usually about 10 mg/kg body weight or more (e.g., about 10 mg/kg or more, about 15 mg/kg or more, about 20 mg/kg or more, about 25 mg/kg or more, about 30 mg/kg or more, about 35 mg/kg or more, about 40 mg/kg or more, about 45 mg/kg or more, about 50 mg/kg or more, or about 55 mg/kg or more, or about 60 mg/kg or more, or about 65 mg/kg or more, or about 70 mg/kg or more), or from about 10 mg/kg to about 70 mg/kg (e.g., from about 10 mg/kg to about 67.5 mg/kg, or from about 10 mg/kg to about 60 mg/kg).

In some embodiments, the therapeutically effective amount of the anti-PD-L1 antibody is 10 mg/kg. In some embodiments, the anti-PD-L1 antibody is administered every 14 days. In some embodiments, the anti-PD-L1 antibody is administered 7 days after the priming dose and every 14 days thereafter. In some embodiments, the anti-PD-L1 antibody is administered on the same day as the priming dose and every 14 days thereafter.

The dose required to achieve and/or maintain a particular serum level of the administered composition is proportional to the amount of time between doses and inversely proportional to the number of doses administered. Thus, as the frequency of dosing increases, the required dose decreases. The optimization of dosing strategies will be readily understood and practiced by one of ordinary skill in the art. An exemplary treatment regime entails administration once every two weeks or once a month or once every 3 to 6 months. Therapeutic entities of the present invention are usually administered on multiple occasions. Intervals between single dosages can be weekly, monthly or yearly. Intervals can also be irregular as indicated by measuring blood levels of the therapeutic entity in the patient. Alternatively, therapeutic entities of the present invention can be administered as a sustained release formulation, in which case less frequent administration is required. Dosage and frequency vary depending on the half-life of the polypeptide in the patient.

A "maintenance dose" is a dose intended to be a therapeutically effective dose. For example, in experiments to determine the therapeutically effective dose, multiple different maintenance doses may be administered to different subjects. As such, some of the maintenance doses may be therapeutically effective doses and others may be sub-therapeutic doses.

In prophylactic applications, a relatively low dosage may be administered at relatively infrequent intervals over a long period of time. Some patients continue to receive treatment for the rest of their lives. In other therapeutic applications, a relatively high dosage at relatively short intervals is sometimes required until progression of the disease is reduced or terminated, and preferably until the patient shows partial or complete amelioration of symptoms of disease. Thereafter, the patent can be administered a prophylactic regime.

In still other embodiments, methods of the present invention include treating, reducing or preventing tumor growth, tumor metastasis or tumor invasion of cancers including carcinomas, hematologic cancers, melanomas, sarcomas, gliomas, *etc.* For prophylactic applications, pharmaceutical compositions or medicaments are administered to a patient susceptible to, or otherwise at risk of disease in an amount sufficient to eliminate or reduce the risk, lessen the severity, or delay the outset of the disease, including biochemical, histologic and/or behavioral symptoms of the disease, its complications and intermediate pathological phenotypes presenting during development of the disease.

Toxicity of the combined agents described herein can be determined by standard pharmaceutical procedures in cell cultures or experimental animals, *e.g.,* by determining the LD₅₀ (the dose lethal to 50% of the population) or the LD₁₀₀ (the dose lethal to 100% of the population). The dose ratio between toxic and therapeutic effect is the therapeutic index. The data obtained from these cell culture assays and animal studies can be used in formulating a dosage range that is not toxic for use in human. The dosage of the proteins described herein lies preferably within a range of circulating concentrations that include the effective dose with little or no toxicity. The dosage can vary within this range depending upon the dosage form employed and the route of administration utilized. The exact formulation, route of administration and dosage can be chosen by the individual physician in view of the patient's condition.

### Primer agents and priming dose

In some embodiments of the methods described herein, a primer agent is administered prior to administering a therapeutically effective dose of an anti-CD47 antibody and an anti-PD-L1 antibody, to the individual. Suitable primer agents include an erythropoiesis-stimulating agent (ESA), and/or a priming dose of an anti-CD47 antibody. Following administration of the priming agent, and allowing a period of time effective for an increase in reticulocyte production, a therapeutic dose of an anti-CD47 antibody is administered. Administration may be made in accordance with the methods described in US patent 9,623,079, herein specifically incorporated by reference.

In some embodiments, administration of a combination of agents of the invention is combined with an effective dose of an agent that increases patient hematocrit, for example erythropoietin stimulating agents (ESA). Such agents are known and used in the art, including, for example, Aranesp^{®} (darbepoetin alfa), Epogen^{®}NF/Procrit^{®}NF (epoetin alfa), Omontys^{®} (peginesatide), Procrit^{®}, etc.

The term "priming dose" or as used herein refers to a dose of an anti-CD47 antibody that primes a subject for administration of a therapeutically effective dose of anti-CD47 antibody such that the therapeutically effective dose does not result in a severe loss of RBCs (reduced hematocrit or reduced hemoglobin). The specific appropriate priming dose of an anti-CD47 antibody can vary depending on the nature of the antibody used and on numerous subject-specific factors (e.g., age, weight, etc.). Examples of suitable priming doses of an anti-CD47 antibody include from about 0.5 mg/kg to about 5 mg/kg, from about 0.5 mg/kg to about 4 mg/kg, from about 0.5 mg/kg to about 3 mg/kg, from about 1 mg/kg to about 5 mg/kg, from about 1 mg/kg to about 4 mg/kg, from about 1 mg/kg to about 3 mg/kg, about 1 mg/kg, about 2 mg/kg, about 3 mg/kg, about 4 mg/kg, about 5 mg/kg. In some embodiments, the priming does is preferably 1 mg/kg.

In some embodiments of the methods described herein, the anti-CD47 antibody is administered to the subject as a priming dose ranging from about 0.5 to about 5 mg/kg of antibody, optionally 1 mg/kg of antibody. In some embodiments, the anti-CD47 antibody is administered to the subject as a dose ranging from about 20 to about 67.5 mg/kg of antibody, optionally 20 mg/kg of antibody, 30 mg/kg of antibody, 45 mg/kg of antibody, 60 mg/kg of antibody, or 67.5 mg/kg of antibody.

In some embodiments of the invention, a primer agent is administered prior to administering a therapeutically effective dose of an anti-CD47 antibody to the individual. Suitable primer agents include an erythropoiesis-stimulating agent (ESA), and/or a priming dose of an anti-CD47 antibody. Following administration of the priming agent, and allowing a period of time effective for an increase in reticulocyte production, a therapeutic dose of an anti-CD47 antibody is administered. The therapeutic dose can be administered in number of different ways. In some embodiments, two or more therapeutically effective doses are administered after a primer antibody is administered. In some embodiments a therapeutically effective dose of an anti-CD47 antibody is administered as two or more doses of escalating concentration, in others the doses are equivalent.

In some embodiments of the invention, an effective priming dose of Hu-5F9G4 is provided, where the effective priming dose for a human is around about 1 mg/kg, e.g. from at least about 0.5 mg/kg up to not more than about 5 mg/kg; from at least about 0.75 mg/kg up to not more than about 1.25 mg/kg; from at least about 0.95 mg/kg up to not more than about 1.05 mg/kg; and may be around about 1 mg/kg

In some embodiments of the invention, an initial dose of an anti-CD47 antibody is infused over a period of at least about 2 hours, at least about 2.5 hours, at least about 3 hours, at least about 3.5 hours, at least about 4 hours, at least about 4.5 hours, at least about 5 hours, at least about 6 hours or more. In some embodiments an initial dose is infused over a period of time from about 2.5 hours to about 6 hours; for example from about 3 hours to about 4 hours. In some such embodiments, the dose of antibody in the infusate is from about 0.05 mg/ml to about 0.5 mg/ml; for example from about 0.1 mg/ml to about 0.25 mg/ml.

In some embodiments a priming dose may be delivered through a sub-cutaneous route, by injection, patch, osmotic pump, and the like as known in the art.

Following administration of the priming antibody, and allowing a period of time effective for an increase in reticulocyte production, a therapeutic dose of an anti-CD47 antibody is administered. The therapeutic dose can be administered in number of different ways. In some embodiments, two or more therapeutically effective doses are administered after a primer agent is administered, e.g. in a weekly dosing schedule. In some embodiments a therapeutically effective dose of an anti-CD47 antibody is administered as two or more doses of escalating concentration, in others the doses are equivalent.

In other embodiments, an initial dose of a CD47 binding antibody, e.g. a priming dose, is administered by continuous fusion, e.g. as an osmotic pump, delivery patch, etc., where the dose is administered over a period of at least about 6 hours, at least about 12 hours, at least about 24 hours, at least about 2 days, at least about 3 days. Many such systems are known in the art. For example DUROS technology, provides a bi-compartment system separated by a piston. One of the compartments consists of osmotic engine specifically formulated with an excess of solid NaCl, such that it remains present throughout the delivery period and results in a constant osmotic gradient. It also consists of a semi permeable membrane on one end through which water is drawn into the osmotic engine and establishes a large and constant osmotic gradient between the tissue water and the osmotic engine. Other compartment consists of a drug solution with an orifice from which the drug is released due to the osmotic gradient. This helps to provide site specific and systemic drug delivery when implanted in humans. The preferred site of implantation is subcutaneous placement in the inside of the upper arm.

Following administration of the priming agent, and allowing a period of time effective for an increase in reticulocyte production, a therapeutic dose of the anti-CD47 antibody is administered. The therapeutic dose can be administered in number of different ways. In some embodiments, two or more therapeutically effective doses are administered after a primer agent is administered, e.g. in a weekly dosing schedule. In some embodiments a therapeutically effective dose of the anti-CD47 antibody is administered as two or more doses of escalating concentration, in others the doses are equivalent. There is reduced hemagglutination after the priming dose, and therefore the extended infusion time is not required.

### Administration

In the methods described herein, compositions, e.g., an anti-CD47 antibody and an anti PD-L1 antibody, are administered to a subject. The compositions can be administered by parenteral, topical, intravenous, intra-abdominal, intratumoral, oral, subcutaneous, intraarterial, intracranial, intraperitoneal, intranasal or intramuscular means. A typical route of administration is intravenous or intratumoral, although other routes can be equally effective.

In some embodiments the anti-CD47 antibody and/or the anti PD-L1 antibody is administered intra-abdominally. In some embodiments the anti-CD47 antibody and/or the anti PD-L1 antibody is administered intravenously. In some embodiments the anti-CD47 antibody and/or the anti PD-L1 antibody is administered intra-tumorally. In one embodiment, a priming dose of the anti-CD47 antibody is administered, and the priming dose is delivered subcutaneously. In some embodiments, the anti-CD47 antibody and the anti PD-L1 antibody are administered concurrently. In some embodiments, the anti-CD47 antibody and the anti PD-L1 antibody are administered sequentially.

The active agents are administered within a period of time to produce an additive or synergistic effect on depletion of cancer cells in the host. Methods of administration include, without limitation, systemic administration, intra-tumoral administration, etc. Usually the anti-CD47 antibody is administered within about a period of about 45 days, about 30 days, about 21 days, about 14 days, about 10 days, about 8 days, about 7 days, about 6 days, about 5 days, about 4 days, about 3 days, about 2 days, about 1 day or substantially the same day as the anti PD-L1 antibody. In some embodiments the anti-CD47 antibody is administered prior to the anti PD-L1 antibody. In some embodiments the anti-CD47 antibody is administered after the anti PD-L1 antibody. The agents can be considered to be combined if administration scheduling is such that the serum level of both agents is at a therapeutic level at the same time. Administration may be repeated as necessary for depletion of the cancer cell population.

### Pharmaceutical Compositions

The methods described herein include administration of pharmaceutical compositions comprising the anti-CD47 antibody and/or the anti PD-L1 antibody.

Typically, the compositions are prepared as injectables, either as liquid solutions or suspensions; solid forms suitable for solution in, or suspension in, liquid vehicles prior to injection can also be prepared. The preparation also can be emulsified or encapsulated in liposomes or micro particles such as polylactide, polyglycolide, or copolymer for enhanced adjuvant effect, as discussed above. Langer, Science 249: 1527, 1990 and Hanes, Advanced Drug Delivery Reviews 28: 97-119, 1997. The agents of this invention can be administered in the form of a depot injection or implant preparation which can be formulated in such a manner as to permit a sustained or pulsatile release of the active ingredient. The pharmaceutical compositions are generally formulated as sterile, substantially isotonic and in full compliance with all Good Manufacturing Practice (GMP) regulations of the U.S. Food and Drug Administration.

The pharmaceutical compositions can be administered in a variety of unit dosage forms depending upon the method of administration. For example, unit dosage forms suitable for oral administration include, but are not limited to, powder, tablets, pills, capsules and lozenges. It is recognized that compositions of the invention when administered orally, should be protected from digestion. This is typically accomplished either by complexing the molecules with a composition to render them resistant to acidic and enzymatic hydrolysis, or by packaging the molecules in an appropriately resistant carrier, such as a liposome or a protection barrier. Means of protecting agents from digestion are well known in the art.

The compositions for administration will commonly comprise an antibody or other ablative agent dissolved in a pharmaceutically acceptable carrier, preferably an aqueous carrier. A variety of aqueous carriers can be used, e.g., buffered saline and the like. These solutions are sterile and generally free of undesirable matter. These compositions may be sterilized by conventional, well known sterilization techniques. The compositions may contain pharmaceutically acceptable auxiliary substances as required to approximate physiological conditions such as pH adjusting and buffering agents, toxicity adjusting agents and the like, e.g., sodium acetate, sodium chloride, potassium chloride, calcium chloride, sodium lactate and the like. The concentration of active agent in these formulations can vary widely, and will be selected primarily based on fluid volumes, viscosities, body weight and the like in accordance with the particular mode of administration selected and the patient's needs (e.g., Remington's Pharmaceutical Science (15th ed., 1980) and Goodman & Gillman, The Pharmacological Basis of Therapeutics (Hardman et al., eds., 1996)).

"Pharmaceutically acceptable excipient" means an excipient that is useful in preparing a pharmaceutical composition that is generally safe, non-toxic, and desirable, and includes excipients that are acceptable for veterinary use as well as for human pharmaceutical use. Such excipients can be solid, liquid, semisolid, or, in the case of an aerosol composition, gaseous.

"Pharmaceutically acceptable salts and esters" means salts and esters that are pharmaceutically acceptable and have the desired pharmacological properties. Such salts include salts that can be formed where acidic protons present in the compounds are capable of reacting with inorganic or organic bases. Suitable inorganic salts include those formed with the alkali metals, *e.g.* sodium and potassium, magnesium, calcium, and aluminum. Suitable organic salts include those formed with organic bases such as the amine bases, *e.g.,* ethanolamine, diethanolamine, triethanolamine, tromethamine, N methylglucamine, and the like. Such salts also include acid addition salts formed with inorganic acids (*e.g.,* hydrochloric and hydrobromic acids) and organic acids (*e.g.,* acetic acid, citric acid, maleic acid, and the alkane- and arene-sulfonic acids such as methanesulfonic acid and benzenesulfonic acid). Pharmaceutically acceptable esters include esters formed from carboxy, sulfonyloxy, and phosphonoxy groups present in the compounds, *e.g.,* C₁₋₆ alkyl esters. When there are two acidic groups present, a pharmaceutically acceptable salt or ester can be a mono-acid-mono-salt or ester or a di-salt or ester; and similarly where there are more than two acidic groups present, some or all of such groups can be salified or esterified. Compounds named in this invention can be present in unsalified or unesterified form, or in salified and/or esterified form, and the naming of such compounds is intended to include both the original (unsalified and unesterified) compound and its pharmaceutically acceptable salts and esters. Also, certain compounds named in this invention may be present in more than one stereoisomeric form, and the naming of such compounds is intended to include all single stereoisomers and all mixtures (whether racemic or otherwise) of such stereoisomers.

The terms "pharmaceutically acceptable", "physiologically tolerable" and grammatical variations thereof, as they refer to compositions, carriers, diluents and reagents, are used interchangeably and represent that the materials are capable of administration to or upon a human without the production of undesirable physiological effects to a degree that would prohibit administration of the composition.

### Kits

Also described herein are kits comprising the active agents, e.g., an anti-CD47 antibody and an anti-PD-L1 antibody, and formulations thereof, and instructions for use. The kit can further contain a least one additional reagent, e.g. a chemotherapeutic drug, ESA, *etc.* Kits typically include a label indicating the intended use of the contents of the kit. The term label includes any writing, or recorded material supplied on or with the kit, or which otherwise accompanies the kit.

### Sequences

In some embodiments, the methods described herein include administration of antibodies with sequences described herein; e.g., the heavy chain, light chain, and/or CDR sequences described herein. The sequences of the administered antibodies can be, e.g., at least 95, 96, 97, 98, 99, or 100% identical to the sequences described herein.

The term percent "identity," in the context of two or more nucleic acid or polypeptide sequences, refer to two or more sequences or subsequences that have a specified percentage of nucleotides or amino acid residues that are the same, when compared and aligned for maximum correspondence, as measured using one of the sequence comparison algorithms described below (e.g., BLASTP and BLASTN or other algorithms available to persons of skill) or by visual inspection. Depending on the application, the percent "identity" can exist over a region of the sequence being compared, e.g., over a functional domain, or, alternatively, exist over the full length of the two sequences to be compared.

For sequence comparison, typically one sequence acts as a reference sequence to which test sequences are compared. When using a sequence comparison algorithm, test and reference sequences are input into a computer, subsequence coordinates are designated, if necessary, and sequence algorithm program parameters are designated. The sequence comparison algorithm then calculates the percent sequence identity for the test sequence(s) relative to the reference sequence, based on the designated program parameters.

Optimal alignment of sequences for comparison can be conducted, e.g., by the local homology algorithm of Smith & Waterman, Adv. Appl. Math. 2:482 (1981), by the homology alignment algorithm of Needleman & Wunsch, J. Mol. Biol. 48:443 (1970), by the search for similarity method of Pearson & Lipman, Proc. Nat'l. Acad. Sci. USA 85:2444 (1988), by computerized implementations of these algorithms (GAP, BESTFIT, FASTA, and TFASTA in the Wisconsin Genetics Software Package, Genetics Computer Group, 575 Science Dr., Madison, Wis.), or by visual inspection (see generally Ausubel et al., infra).

One example of an algorithm that is suitable for determining percent sequence identity and sequence similarity is the BLAST algorithm, which is described in Altschul et al., J. Mol. Biol. 215:403-410 (1990). Software for performing BLAST analyses is publicly available through the National Center for Biotechnology Information (www.ncbi.nlm.nih.gov/).

### EXAMPLES

### Example 1

### In vitro synergy experiment

An ADCC assay is performed using mouse or human NK cells (effectors) and mouse or human cancer cells (target cells). Mouse NK cells are isolated from peripheral blood, bone marrow, or spleens; human NK cells are isolated from peripheral blood. Human cancer cell lines or primary samples are labeled for use as target cells (e.g. with chromium or fluorescent dye).

The NK cells and cancer cells are combined in vitro, and co-culture with the following treatments:
- Vehicle control (e.g. PBS)
- Anti-PD-L1 antibody alone, including avelumab
- Anti-CD47 antibody alone
- Anti-CD47 antibody plus anti-PD-L1 antibody
   ADCC is measured via chromium-release assay or flow cytometry cell death assays (e.g., Annexin V/DAPI staining). NK cell cytokine (e.g. IFN-gamma) release is measured via ELISA. The change in cell death and cytokine release in the presence of checkpoint inhibitor combined with anti-CD47 is determined relative to the mono-therapies listed above.

### Example 2

### In vivo experiment protocol

Cancer cells are injected into mice via subcutaneous, retroperitoneal, or peripheral blood injection and allowed to engraft. The animals are randomized into four treatment groups:
- Vehicle control (e.g. PBS)
- Anti-PD-L1 antibody alone, including avelumab
- Anti-CD47 antibody alone
- Anti-CD47 antibody plus anti-PD-L1 antibody

Mice are treated daily, three times per week, twice per week, or once per week with the respective treatments. Tumor burden is measured by tumor volume measurements, bioluminescence using labeled cancer cells (e.g. luciferase positive cells), and/or analysis of peripheral blood. The overall survival of the mice is also measured.

### Example 3

### A Study Of Avelumab In Combination With anti-CD47 Antibody In Ovarian Cancers

This is dose-optimization study to evaluate safety, pharmacokinetics, pharmacodynamics, and antitumor activity of avelumab (MSB0010718C) in combination with CD47 blockade in solid tumor patients and checkpoint-naive ovarian, fallopian tube cancer, and primary peritoneal carcinoma patients who have previously progressed within 6 months of prior platinum chemotherapy. The primary purpose is to assess the safety and efficacy of various combinations with CD47 blockade, optimizing dosing regimens as appropriate, in a limited series of indications. Initially, the study will evaluate the safety and antitumor activity of avelumab, an anti-PD-L1 monoclonal antibody (mAb) in combination with 5F9-G4, a humanized antibody that blocks interactions between CD47 and SIRPalpha. Secondary objectives include determining a recommended dose of Hu5F9-G4 + avelumab in solid tumor patients, examining the pharmacokinetic (PK) and pharmacodynamic (PD) profiles of Hu5F9-G4 in combination with avelumab, evaluating the immunogenicity of Hu5F9-G4 in combination with avelumab, and evaluating the impact of this combination on the myeloid cell populations in the tumor microenvironment as assessed in sequential tumor biopsies in patients with platinum-resistant ovarian cancer.

Primary Objective Measures: Number of participants with Dose-Limiting Toxicities (DLT) will be monitored for the first 5 weeks on treatment, starting with the initial priming dose of Hu5F9-G4. The first cycle will be 5 weeks in duration and subsequent cycles will be every 4 weeks. Initial tumor response assessments will be made just prior to week 10 of treatment (prior to cycle 3) and then after every 8 weeks of treatment (every 2 cycles). All toxicities will be graded according to the NCI CTCAE Version 4.03. A DLT is defined as any Grade 3 or greater AE that is assessed as related to study treatment that occurs during the 4-week DLT observation period.

Secondary Objective Measures: In the ovarian cancer cohort, tumor biopsies will be obtained where medically feasible in all patients at baseline during screening and after at the end of cycle 2 (± 2 weeks). Tumor biopsies are optional for the safety run-in patients. Screening will last up to 30 days before first dose of study drug, during which time the patient's eligibility and baseline characteristics will be determined. Efficacy will be evaluated using iRECIST, criteria. Treatment with study drug may be continued until an unacceptable drug related toxicity occurs or until disease progression by iRECIST. Patients who experience initial disease progression can remain on study until they are deemed to have progressive disease by iRECIST provided all of the following are conditions are met: absence of worsening symptoms from their tumor, no unacceptable or irreversible toxicities related to study treatment, no evidence of clinical deterioration or declining performance status, and no impending life threatening complications arising from tumor growth. Post treatment, patients will be observed for survival until death, withdrawal of consent, or the end of the study, whichever occurs first.

**Table 2.**

| **Dose Cohort** | **Drug/Dose (IV)** | **Dose Schedule** | |
|---|---|---|---|
| | | **Cycle 1 (35 days)** | **Cycle 2+ (28 days)** |
| -1 | Hu5F9-G4 1 mg/kg (prime) | Day 1 | - |
| | Hu5F9-G4 20 mg/kg (maintenance) | Day 8, 15, 22, 29 | Day 1, 8, 15, 22 |
| | Avelumab 10 mg/kg every 2 weeks | Day 8, 22 | Day 1, 15 |
| 1 (starting dose) | Hu5F9-G4 1 mg/kg (prime) | Day 1 | - |
| | Hu5F9-G4 30 mg/kg (maintenance) | Day 8, 15, 22, 29 | Day 1, 8, 15, 22 |
| | Avelumab 10 mg/kg every 2 weeks | Day 8, 22 | Day 1, 15 |

### Example 4

### T cell Assays

Antigen presentation assay. For *in vitro* antigen presentation assays, 10⁴ macrophages are co-cultured with equal numbers of DLD1-cOVA-GFP cancer cells overnight in serum-free RPMI media. The following day, equal volume of RPMI + 20% FCS is added to the cultures. Peripheral lymph nodes are harvested from OT-I or OT-II TCR transgenic mice and labeled with 0.5 mM CFSE (Molecular Probes). T cells are isolated using biotinylated anti-CD8 or anti-CD4 antibodies, followed by enrichment with anti-biotin magnetic beads (Miltenyi Biotec). 5 × 10⁴ T cells are added to the cultures and analyzed at day 3 (for OT-I T cells) or day 4 (for OT-II T cells). For in vivo antigen presentation assays, 2 × 10⁶ CFSE-labeled OT-I T cells (CD45.2) are adoptively transferred iv into recipient mice (CD45.1). Macrophages are isolated from co-culture with cancer cells and injected into the footpad of mice. Popliteal lymph nodes are analyzed on day 4 for CFSE dilution within CD45.2+ cells.

In vivo cell killing assay. In brief, splenocytes from C57BL/Ka (CD45.1) mice are labeled with 10 uM CFSE (CFSE-high) and 1 uM CFSE (CFSE-low). CFSE-high splenocytes are pulsed in a 6-well plate with 1 uM SIINFEKL peptide (SEQ ID NO: 5) for 1 hour. Cells are mixed in a 1:1 ratio with non-peptide-pulsed CFSE-low cells before iv transfer. To account for variation in the CFSE high/low ratio in the absence of peptide-specific lysis, control mice receive CFSE-high splenocytes not pulsed with SIINFEKL peptide (SEQ ID NO: 5) before mixing in a 1:1 ratio with CFSE-low splenocytes and transfer to mice. Draining lymph nodes are analyzed 16 hours later. Percent cytotoxicity was calculated as (1 - %CFSE^{high}/%CFSE^{low}) normalized to the ratio in control mice receiving splenocytes not pulsed with SIINFEKL peptide (SEQ ID NO: 5).

Tumor challenge. 1 × 10⁶ CD8-enriched OT-I T cells are adoptively transferred iv into recipient C57BL/Ka mice. Macrophages from syngeneic C57BL/Ka mice are co-cultured with DLD1-cOVA-GFP cancer, and then isolated by magnetic enrichment and injected into the footpad of mice. The tumor cell line E.G7 (EL.4 cells expressing the chicken OVA cDNA) is used for tumor challenge of mice (ATCC). 1 × 105 E.G7 cells are injected s.c. into the right hindlimb of the mice in a 1:1 ratio with regular matrigel. Tumor size is measured every day by using fine calipers and volume calculated based on length ^{∗} width ^{∗} height ^{∗} π/6.

T Cell Proliferation. Mature T cells recognize and respond to the antigen/MHC complex through their antigen-specific receptors (TCR). The most immediate consequence of TCR activation is the initiation of signaling pathways including induction of specific protein tyrosine kinases (PTKs), breakdown of phosphatidylinositol 4,5-biphosphate (PIP2), activation of protein kinase C (PKC) and elevation of intracellular calcium ion concentration. These early events are transmitted to the nucleus and result in clonal expansion of T cells; upregulation of activation markers on the cell surface; differentiation into effector cells; induction of cytotoxicity or cytokine secretion; induction of apoptosis.

T cell activation is assessed by measuring T cell proliferation upon in vitro stimulation of T cells via antigen or agonistic antibodies to TCR. This protocol is written as a starting point for examining in vitro proliferation of mouse splenic T-cells and human peripheral T cells stimulated via CD3. Critical parameters include cell density, antibody titer and activation kinetics.

Prepare a 5-10 µg/mL solution of anti-CD3e (145-2C11) in sterile PBS. Calculate the number of wells required for each experimental condition and consider triplicate samples for each condition. For example, to coat one-half plate (48 wells) 2.6mL of antibody solution is required. Dispense 50 µL of the antibody solution to each well of the 96-well assay plate. For the control unstimulated wells, add 50 µL of sterile PBS. Tightly cover the plate with ParafilmTM to avoid sample evaporation and incubate at 37°C for 2 hours or prepare the plate one day in advance and keep at 4°C overnight. Just before adding cells, remove the 50 µL antibody solution with a multichannel pipettor. Rinse each well with 200 µL of sterile PBS and discard PBS.

Harvest spleen and prepare a single cell suspension under sterile conditions and resuspend in complete RPMI-1640 at 10⁶/mL in the presence of the desired agents, e.g. anti-CD47, checkpoint inhibitors, etc. Add 200 µL of the cell suspension to each well and place in a humidified 37°C, 5% CO2 incubator. Add soluble anti-CD28 to cells at 2 ug/mL. Incubate for 2-4 days. Cells can be harvested and processed for quantitation.

Each publication cited in this specification is hereby incorporated by reference in its entirety for all purposes.

It is to be understood that this invention is not limited to the particular methodology, protocols, cell lines, animal species or genera, and reagents described, as such may vary. It is also to be understood that the terminology used herein is for the purpose of describing particular embodiments only, and is not intended to limit the scope of the present invention, which will be limited only by the appended claims

As used herein the singular forms "a", "and", and "the" include plural referents unless the context clearly dictates otherwise. Thus, for example, reference to "a cell" includes a plurality of such cells and reference to "the culture" includes reference to one or more cultures and equivalents thereof known to those skilled in the art, and so forth. All technical and scientific terms used herein have the same meaning as commonly understood to one of ordinary skill in the art to which this invention belongs unless clearly indicated otherwise.

The invention further provides the following numbered embodiments:
1. A method of treating a human subject having epithelial ovarian cancer, comprising
   a. administering a priming dose of Hu5F9-G4 antibody to the subject, wherein the priming dose is 1 mg/kg of Hu5F9-G4 antibody; and
   b. administering a therapeutically effective dose of Hu5F9-G4 antibody to the subject, wherein the therapeutically effective dose of Hu5F9-G4 antibody is 20 to 60 mg/kg, and wherein step (b) is performed after at least about 7 days after beginning step (a) and every 7 days thereafter; and
   c. administering Avelumab to the subject, wherein the dose of Avelumab is 10 mg/kg, and wherein step (c) is performed at least about 7 days after step (a) and every 14 days thereafter.
2. A method of treating a human subject having epithelial ovarian cancer, comprising
   a. administering a priming dose of Hu5F9-G4 antibody to the subject, wherein the priming dose is 1 mg/kg of Hu5F9-G4 antibody; and
   b. administering a therapeutically effective dose of Hu5F9-G4 antibody to the subject, wherein the therapeutically effective dose of Hu5F9-G4 antibody is 30 mg/kg, and wherein step (b) is performed after at least about 7 days after beginning step (a) and every 7 days thereafter; and
   c. administering Avelumab to the subject, wherein the dose of Avelumab is 10 mg/kg, and wherein step (c) is performed at least about 7 days after step (a) and every 14 days thereafter.
3. A method of treating a human subject having an ovarian cancer or reducing the size of the ovarian cancer in the subject, comprising administering: a therapeutically effective amount of an anti-CD47 antibody to the subject; and a therapeutically effective amount of at least one anti-PD-L1 antibody to the subject.
4. The method of embodiment 3, wherein the ovarian cancer is an epithelial ovarian cancer, optionally serous tumor, mucinous tumor, clear cell tumor, endometriod tumor, transitional cell tumor, Brenner tumor, carcinosarcoma tumor, mixed epithelial tumor, borderline epithelial tumor, undifferentiated carcinoma tumor, fallopian tube tumor, or primary peritoneal tumor.
5. The method of embodiment 4, wherein the epithelial ovarian cancer is serous tumor.
6. The method of embodiment 5, wherein the serous tumor ovarian cancer is low grade or high grade as determined by histological analysis subtyping.
7. The method of any of the above embodiments, wherein the tumor type is determined by histological analysis.
8. The method of any of the above embodiments, wherein the subject is anti-PD-L1 antibody naive.
9. The method of any of the above embodiments, wherein the anti-CD47 antibody and the anti-PD-L1 antibody are administered concurrently or sequentially.
10. The method of any of the above embodiments, wherein the anti-CD47 antibody comprises an IgG4 Fc.
11. The method of any of the above embodiments, wherein the anti-CD47 antibody competes for binding to CD47 with Hu5F9-G4.
12. The method of any of the above embodiments, wherein the anti-CD47 binds to the same CD47 epitope as Hu5F9-G4.
13. The method of any of the above embodiments, wherein the anti-CD47 antibody is Hu5F9-G4.
14. The method of any of the above embodiments, wherein the anti PD-L1 antibody is Avelumab (Bavencio ^{®}).
15. The method of any of the above embodiments, wherein the anti-CD47 antibody is Hu5F9-G4 and the anti PD-L1 antibody is Avelumab (Bavencio ^{®}).
16. The method of any of the above embodiments, wherein the anti-CD47 antibody and the anti-PD-L1 antibody are each formulated in a pharmaceutical composition with a pharmaceutically acceptable excipient.
17. The method of any of the above embodiments, wherein the human subject is platinum sensitive.
18. The method of any of the above embodiments except embodiment 15, wherein the human subject is platinum resistant.
19. The method of any of the above embodiments, wherein the anti-CD47 antibody and/or the anti PD-L1 antibody is administered intravenously.
20. The method of any of the above embodiments, wherein the anti-CD47 antibody and/or the anti PD-L1 antibody is administered intra-abdominally.
21. The method of any of the above embodiments, wherein the anti-CD47 antibody and/or anti-PD-L1 antibody is administered intra-tumorally.
22. The method of any of the above embodiments, wherein administration reduces the level of CA125 in the subject compared to baseline, optionally wherein the level of CA125 is measured about once per month.
23. The method of any of the above embodiments, wherein administration reduces the level of CA125 in the subject by at least 30-90, 40-80, 50-70, 30, 40, 50, 60, 70, 80, or 90% compared to baseline.
24. The method of any of the above embodiments, wherein administration reduces the size of the cancer or metastases thereof compared to baseline, optionally as measured by imaging, optionally wherein the imaging is CT/PET/CT or MRI, optionally comprising disease that increases initially from baseline but subsequently decreases in size.
25. The method of any of the above embodiments, wherein administration reduces the level of at least one of CA125, HE4 (human epididymis protein 4), CA-72-4, CA-19-9, and CEA; compared to baseline.
26. The method of any of the above embodiments, further comprising administering a priming dose of the anti-CD47 antibody.
27. The method of any of the above embodiments, further comprising administering a priming dose of an erythropoietin stimulating agent.
28. The method of any of embodiment 26, wherein the anti-CD47 antibody is administered to the subject as a priming dose ranging from about 0.5 to about 5 mg/kg of antibody, optionally 1 mg/kg of antibody.
29. The method of any of the above embodiments, wherein the anti-CD47 antibody is administered to the subject as a dose ranging from about 20 to about 67.5 mg/kg of antibody, optionally 20 mg/kg of antibody, 30 mg/kg of antibody, 45 mg/kg of antibody, 60 mg/kg of antibody, or 67.5 mg/kg of antibody.
30. The method of any of the above embodiments, wherein the anti-CD47 antibody is administered to the subject weekly, every 2 weeks, or every 3 weeks.
31. The method of any of the above embodiments, wherein the method comprises:
   a. administering a priming dose of the anti-CD47 antibody to the subject, wherein the priming dose is from about 0.5 to about 5 mg/kg of antibody; and
   b. administering a therapeutically effective dose of the anti-CD47 antibody to the subject, wherein step (b) is performed after at least about 3 to 14 days after beginning step (a), optionally being 7 days after (a).
32. The method of embodiment 31, wherein the method comprises (a) administering the priming dose of anti-CD47 antibody to the subject at a dose of 1 mg/kg of antibody on day 1; and (b) administering the therapeutically effective dose of the anti-CD47 antibody to the subject at a dose of 20 mg/kg of antibody, 30 mg/kg of antibody, 45 mg/kg of antibody, 60 mg/kg of antibody, or 67.5 mg/kg of antibody on day 8.
33. The method of any of embodiments 26-32, wherein the effectiveness of the priming dose is determined based on the anemia status of the subject following administration of the priming dose.
34. The method of any of embodiments 26-32, wherein the priming dose is considered effective if: the fall in the subject's hemoglobulin level is not less than 8.0 g/dL; and/or the absolute fall in the subject's hemoglobin level is less than 3.0 to 3.75 g/dL.
35. The method of embodiment 31, further comprising after step (a) and prior to step (b): a step of determining whether administration of the priming dose was effective.
36. The method of embodiment 35, wherein the determining step comprises performing a reticulocyte count, wherein administration of the priming dose is determined to have been effective if the reticulocyte count is from about 100 × 10⁹ reticulocytes per L to about - 1000 × 10⁹ reticulocytes per L.
37. The method of embodiment 36, wherein the determining step comprises performing a reticulocyte count, wherein administration of the priming dose is determined to have been effective if the percentage of reticulocytes in the blood is greater than about 1.5%.
38. The method of embodiment 36, wherein the determining step comprises performing a reticulocyte count, wherein administration of the primer agent is determined to have been effective if the reticulocyte index is greater than about 2%.
39. The method of any of embodiments 31-38, wherein the priming dose is administered to the human subject in an infusate with a concentration of from about 0.05 mg/ml to about 0.5 mg/ml of anti-CD47 antibody.
40. The method of embodiment 39, wherein the infusate is delivered over of a period of at least about 1-3, 8-10, 1, 2, 3, 4, 5, 6, 7, 8, 9, or 10 hour(s).
41. The method of embodiment 39, wherein the infusate is delivered over a period of at least about 3 hours.
42. The method of embodiment 39, wherein the infusate is delivered over a period of from about 2.5 hours to about 6 hours.
43. The method of any of embodiments 31-38, where the priming dose is delivered by continuous pump over a period of from about 6 hours to about 3 days.
44. The method of any one of embodiments 31-43, wherein the priming dose is delivered subcutaneously.
45. The method of any one of embodiments 31-44, wherein the priming dose saturates at least about 50% to 100% of CD47 sites on red blood cells, optionally 100% of CD47 sites on red blood cells.
46. The method of embodiment 45, wherein the dose is determined by a receptor occupancy assay, in which following administration of a dose of unlabeled anti-CD47 antibody to the subject, a blood sample is obtained and combined with a saturating dose of detectably labeled anti-CD47 antibody; and determining the level of binding.
47. The method of any of embodiments 31-46, wherein the therapeutically effective dose of (b) is sufficient to achieve a circulating level of greater than 100, 250, 500, or 1000 µg/ml of the anti-CD47 antibody for a sustained period of time, optionally wherein the sustained period of time is at least 1-28, 7-28, 7-21, 14-28, or 21-28 days.
48. The method of embodiment 47, wherein the sustained period of time is from about 1, 2, 3, or 4 weeks.
49. The method of embodiment 31-48, wherein the priming dose is 1 mg/kg of anti-CD47 antibody.
50. The method of embodiment 31-48, wherein the therapeutically effective dose of the anti-CD47 antibody is 20 mg/kg.
51. The method of embodiment 31-48, wherein the therapeutically effective dose of the anti-CD47 antibody is 30 mg/kg.
52. The method of embodiment 31-48, wherein the therapeutically effective dose of the anti-CD47 antibody is 45 mg/kg.
53. The method of embodiment 31-48, wherein the therapeutically effective dose of the anti-CD47 antibody is 60 mg/kg.
54. The method of embodiment 31-48, wherein the therapeutically effective dose of the anti-CD47 antibody is 67.5 mg/kg.
55. The method of any of embodiments 31-54, wherein the therapeutically effective dose of anti-CD47 antibody is administered from about every 7, 14, 21, or 28 days.
56. The method of any of embodiments 31-55, wherein the therapeutically effective dose of the anti-CD47 antibody is administered every 7 days.
57. The method of any of the above embodiments, wherein the therapeutically effective amount of the anti-PD-L1 antibody is 10 mg/kg.
58. The method of embodiment 57, wherein the anti-PD-L1 antibody is administered every 14 days.
59. A composition comprising an anti-CD47 antibody and an anti-PD-L1 antibody.
60. A kit comprising an anti-CD47 antibody, an anti-PD-L1 antibody, and instructions for use.

## Claims

1. A monoclonal anti-CD47 antibody for use in a method of treating a platinum resistant human ovarian cancer, comprising
a. administering a priming dose of the anti-CD47 antibody to the subject, wherein the priming dose is 1 mg/kg of the anti-CD47 antibody; and
b. administering a therapeutically effective dose of the anti-CD47 antibody to the subject, wherein the therapeutically effective dose of the anti-CD47 antibody is 20 to 60 mg/kg, and wherein step (b) is performed after at least about 7 days after beginning step (a) and every 7 days thereafter; and
c. administering Avelumab to the subject, wherein the dose of Avelumab is 10 mg/kg, and wherein step (c) is performed at least about 7 days after step (a) and every 14 days thereafter,
wherein the anti-CD47 antibody has the heavy chain and light chain sequences of
SEQ ID NOs: 1 and 2, respectively.

2. The anti-CD47 antibody for use of claim 1, wherein the therapeutically effective dose of the anti-CD47 antibody is 30 mg/kg

3. The anti-CD47 antibody for use of claim 1 or claim 2, wherein the ovarian cancer is epithelial, optionally wherein the epithelial ovarian cancer is serous tumor, mucinous tumor, clear cell tumor, endometriod tumor, transitional cell tumor, Brenner tumor, carcinosarcoma tumor, mixed epithelial tumor, borderline epithelial tumor, undifferentiated carcinoma tumor, fallopian tube tumor, or primary peritoneal tumor.

4. The anti-CD47 antibody for use of claim 3, wherein the epithelial ovarian cancer is serous tumor, optionally wherein the serous tumor ovarian cancer is low grade or high grade as determined by histological analysis subtyping.

5. The anti-CD47 antibody for use of any of the above claims, wherein:
(i) the tumor type is determined by histological analysis, or
(ii) the subject is anti-PD-L1 antibody naïve.

6. The anti-CD47 antibody for use of any of the above claims, wherein:
(i) the anti-CD47 antibody and Avelumab are administered concurrently or sequentially, and/or
(ii) the anti-CD47 antibody and Avelumab are each formulated in a pharmaceutical composition with a pharmaceutically acceptable excipient, and/or
(iii) the anti-CD47 antibody and/or Avelumab is administered intravenously, intra-abdominally, or intra-tumorally.

7. The anti-CD47 antibody for use of any of the above claims, wherein administration:
(i) reduces the level of CA125 in the subject compared to baseline, optionally wherein the level of CA125 is measured about once per month, and/or
(ii) reduces the level of CA125 in the subject by at least 30-90, 40-80, 50-70, 30, 40, 50, 60, 70, 80, or 90% compared to baseline, and/or
(iii) reduces the size of the cancer or metastases thereof compared to baseline, optionally as measured by imaging, optionally wherein the imaging is CT/PET/CT or MRI, optionally comprising disease that increases initially from baseline but subsequently decreases in size, and/or
(iv) reduces the level of at least one of CA125, HE4 (human epididymis protein 4), CA-72-4, CA-19-9, and CEA; compared to baseline.

8. The anti-CD47 antibody for use of any of the above claims, further comprising administering a priming dose of an erythropoietin stimulating agent.

9. The anti-CD47 antibody for use of any of the above claims, wherein the method comprises (a) administering the priming dose of anti-CD47 antibody to the subject at a dose of 1 mg/kg of antibody on day 1; and (b) administering the therapeutically effective dose of the anti-CD47 antibody to the subject at a dose of 20 mg/kg of antibody, 30 mg/kg of antibody, 45 mg/kg of antibody, or 60 mg/kg of antibody of antibody on day 8.

10. The anti-CD47 antibody for use of any of claims 1-9, wherein:
(i) the effectiveness of the priming dose is determined based on the anemia status of the subject following administration of the priming dose, or
(ii) the priming dose is considered effective if: the fall in the subject's hemoglobulin level is not less than 8.0 g/dL; and/or the absolute fall in the subject's hemoglobin level is less than 3.0 to 3.75 g/dL.

11. The anti-CD47 antibody for use of any one of claims 1-9, further comprising after step (a) and prior to step (b): a step of determining whether administration of the priming dose was effective,
optionally wherein the determining step comprises performing a reticulocyte count, wherein administration of the priming dose is determined to have been effective if the reticulocyte count is from about 100 × 10⁹ reticulocytes per L to about - 1000 × 10⁹ reticulocytes per L,
optionally further wherein the determining step comprises performing a reticulocyte count, wherein: (i) administration of the priming dose is determined to have been effective if the percentage of reticulocytes in the blood is greater than about 1.5%, or (ii) administration of the primer agent is determined to have been effective if the reticulocyte index is greater than about 2%.

12. The anti-CD47 antibody for use of any of claims 1-11, wherein the priming dose is administered to the human subject in an infusate with a concentration of from about 0.05 mg/ml to about 0.5 mg/ml of anti-CD47 antibody, optionally wherein the infusate is delivered over of a period of:
(i) at least about 1-3, 8-10, 1, 2, 3, 4, 5, 6, 7, 8, 9, or 10 hour(s),
(ii) at least about 3 hours, or
(iii) from about 2.5 hours to about 6 hours.

13. The anti-CD47 antibody for use of any of claims 1-12, wherein the priming dose:
(i) is delivered by continuous pump over a period of from about 6 hours to about 3 days, and/or
(ii) is delivered subcutaneously, and/or
(iii) saturates at least about 50% to 100% of CD47 sites on red blood cells, optionally 100% of CD47 sites on red blood cells,
optionally wherein the dose is determined by a receptor occupancy assay, in which following administration of a dose of unlabeled anti-CD47 antibody to the subject, a blood sample is obtained and combined with a saturating dose of detectably labeled anti-CD47 antibody; and determining the level of binding.

14. The anti-CD47 antibody for use of any of claims 1-13, wherein the therapeutically effective dose of (b) is sufficient to achieve a circulating level of greater than 100, 250, 500, or 1000 µg/ml of the anti-CD47 antibody for a sustained period of time, optionally wherein the sustained period of time is at least 1-28, 7-28, 7-21, 14-28, or 21-28 days, optionally wherein the sustained period of time is from about 1, 2, 3, or 4 weeks.

15. The anti-CD47 antibody for use of any one of claims 1-14, wherein the therapeutically effective dose of the anti-CD47 antibody is 20 mg/kg, 30 mg/kg, 45 mg/kg, or 60 mg/kg.
